# EUROPEAN PATENT APPLICATION

(11) **EP 4 749 782 A1**
(43) Date of publication of application: **27.05.2026**
(21) Application number: 24895164.2
(22) Date of filing: 06.11.2024
(51) Int. Cl.: H01M 50/188, H01M 50/153, H01M 50/559

(54) **COVER PLATE ASSEMBLY, MICROBATTERY, AND CAPSULE ENDOSCOPE**

(30) Priority: 24.09.2024 CN 202422342373 U
(71) Applicant: Eve Energy Co. Ltd., Huizhou, Guangdong 516000 (CN)
(72) Inventor: LIU, Zhihua, Huizhou, Guangdong 516000 (CN); HUANG, Yucheng, Huizhou, Guangdong 516000 (CN); QIU, Xinbin, Huizhou, Guangdong 516000 (CN); LONG, Feihong, Huizhou, Guangdong 516000 (CN); ZHAO, Ruirui, Huizhou, Guangdong 516000 (CN); ZHANG, Xinjie, Huizhou, Guangdong 516000 (CN)
(74) Representative: Herrero & Asociados, S.L.
(86) International application number: PCT/CN2024/130271
(87) International publication number: WO 2026/065669

(57) **Abstract**

The present disclosure provides a cover plate assembly, a micro battery, and a capsule endoscope. The cover plate assembly includes a cover plate, an electrode post structure, and a sealing ring. The cover plate is configured to cover and seal the opening and in sealed cooperation with a side wall of the opening, thereby forming a seal between the cover plate and the housing. An outer peripheral side wall of the electrode post structure defines a sealing ring accommodation groove. The sealing ring is disposed on the outer peripheral side wall of the electrode post structure and accommodated within the sealing ring accommodation groove; the sealing ring is in sealed cooperation with the sealing ring accommodation groove, thereby enhancing the sealing performance between the sealing ring and the electrode post structure.

## Description

The present application claims the priority of Chinese patent application No. 202422342373.9, filed on September 24, 2024, in the National Intellectual Property Administration of China, the entire contents of which are hereby incorporated by reference in its entirety.

### TECHNICAL FIELD

The present disclosure relates to the technical field of medical devices and equipment, and more specifically to a cover plate assembly, a micro battery, and a capsule endoscope.

### BACKGROUND

Capsule endoscopes, compared to traditional endoscopes, eliminate pain, invasiveness, infection risks, and mechanical injury. The batteries in capsule endoscopes typically employ manganese dioxide (MnO₂) cells, with their terminal covers predominantly adopting a glass-sealed structure. This structure generally includes a base plate, a glass body, and an electrode post. Specifically, the base plate defines a mounting hole passing through its central axis. The electrode post achieves a hermetic seal with the mounting hole via the glass body. A peripheral edge of the base plate is welded hermetically to the inner wall of the battery housing, thereby achieving the encapsulation of the battery.

### Technical problem

However, the processing and precision control of glass-sealed structures are difficult, and leakage is likely to occur due to processing errors. In addition, most electrode posts require slurry coating or membrane pressing processes, resulting in poor high-current pulse performance.

### SUMMARY OF THE DISCLOSURE

**In** a first aspect, the present disclosure provides a cover plate assembly for a micro battery; wherein the micro battery includes a housing, with an accommodation cavity defined inside the housing and having an opening; the cover plate assembly includes: a cover plate, configured to cover and seal the opening and in sealed cooperation with a side wall of the opening; an electrode post structure, passing through the cover plate; wherein an outer peripheral side wall of the electrode post structure defines a sealing ring accommodation groove; and a sealing ring, disposed on the outer peripheral side wall of the electrode post structure and accommodated within the sealing ring accommodation groove; wherein the sealing ring is in sealed cooperation with the sealing ring accommodation groove.

In a second aspect, the present disclosure provides a micro battery, including the cover plate assembly as above, the housing, and a cell structure; wherein the housing includes a bottom wall and a side wall surrounding the bottom wall; the side wall and the bottom wall together define the accommodation cavity with the opening at one end; a diameter of the housing is greater than or equal to 10 mm; the cover plate is configured to cover and seal the opening, and the cell structure is disposed within the accommodation cavity.

In a third aspect, the present disclosure provides a capsule endoscope, including the micro battery as above, an endoscope housing, and a working module; wherein the micro battery and the working module are both disposed inside the endoscope housing, and the micro battery is electrically connected to the working module.

### Beneficial effects of the present disclosure are as followed.

The present disclosure provides a cover plate assembly for a micro battery, which includes a housing, with an accommodation cavity defined inside the housing and having an opening; the cover plate assembly includes a cover plate, an electrode post structure, and a sealing ring, with the cover plate configured to cover the opening and in sealed cooperation with a side wall of the opening, thereby forming a seal between the cover plate and the housing. An outer peripheral side wall of the electrode post structure defines a sealing ring accommodation groove; the sealing ring is disposed on the outer peripheral side wall of the electrode post structure and accommodated within the sealing ring accommodation groove, and forms a sealed fit with the sealing ring accommodation groove, thereby enhancing the sealing performance between the sealing ring and the electrode post structure, and fixing the sealing ring relative to the electrode post structure for easy assembly. An outer peripheral side wall of the sealing ring is in a sealed fit with an inner peripheral side wall of the cover plate, thereby enabling the inner and outer sides of the sealing ring to form good seals with the electrode post structure and the cover plate, respectively, and thus preventing leakage. Additionally, replacing the traditional glass-sealed structure with the sealing ring may reduce manufacturing complexity and precision control requirements.

The present disclosure further provides a micro battery, including the aforementioned cover plate assembly, as well as a housing and a cell structure. The housing includes a bottom wall and a side wall surrounding the bottom wall. The side wall and the bottom wall together define the accommodation cavity with an opening at one end. The diameter of the housing does not exceed 10 mm, thereby minimizing the size of the micro battery. The cover plate is configured to cover and seal the opening, and the cell structure is disposed within the accommodation cavity. In the cover plate assembly, the cover plate is sealed to the housing, and the inner and outer sides of the sealing ring can form a good seal with the electrode post structure and the cover plate, respectively, to prevent leakage. Additionally, replacing the traditional glass-sealed structure with the sealing ring may reduce manufacturing complexity and precision control requirements.

The present disclosure further provides a capsule endoscope, which includes the aforementioned micro battery, an endoscope housing, and a working module. The micro battery and the working module are both disposed inside the endoscope housing, and the micro battery is electrically connected to the working module. The micro battery has a small size, and the cover plate assembly has good sealing performance with the housing, thereby preventing leakage. Additionally, the use of the sealing ring replaces the traditional glass-sealed structure, thereby reducing manufacturing complexity and precision control requirements.

### BRIEF DESCRIPTION OF THE DRAWINGS

FIG. 1 is a structural schematic view of a micro battery according to some embodiments of the present disclosure.
FIG. 2 is a disassembled schematic view of a cover plate assembly according to some embodiments of the present disclosure.

Reference numerals:
100, Housing; 101, Bottom wall; 102, Side wall; 103, Opening; 104, Accommodation cavity;
200, Cover plate; 201, Mounting hole;
300, Electrode post assembly; 301, Sealing ring accommodation groove; 310, First pressure plate; 320, Electrode post body; 330, Second pressure plate;
400, Sealing ring; 401, Cover plate accommodation groove;
500, Positive electrode sheet; 510, Positive electrode tab;
600, Negative electrode sheet; 610, Negative electrode tab;
700, Insulating layer;
800, Insulating spacer.

### DETAILED DESCRIPTION

The battery of the capsule endoscope is typically adopted with a manganese dioxide battery, with the cover plate generally made of a glass-sealed structure. This structure generally includes a base plate, a glass body, and an electrode post. Specifically, the base plate defines a mounting hole passing through its central axis. The electrode post achieves a hermetic seal with the mounting hole via the glass body. A peripheral edge of the base plate is welded hermetically to the inner wall of the battery housing, thereby achieving the encapsulation of the battery. However, the processing and precision control of glass-sealed structures are difficult, and leakage is likely to occur due to processing errors. In addition, most electrode posts require slurry coating or membrane pressing processes, resulting in poor high-current pulse performance.

The embodiments of the present disclosure provide a micro battery to address the issues of high manufacturing complexity and leakage in the glass-sealed structure, as well as the poor performance of electrode posts under high current pulses, which are common in the related art. The micro battery is applicable to the field of medical device technology.

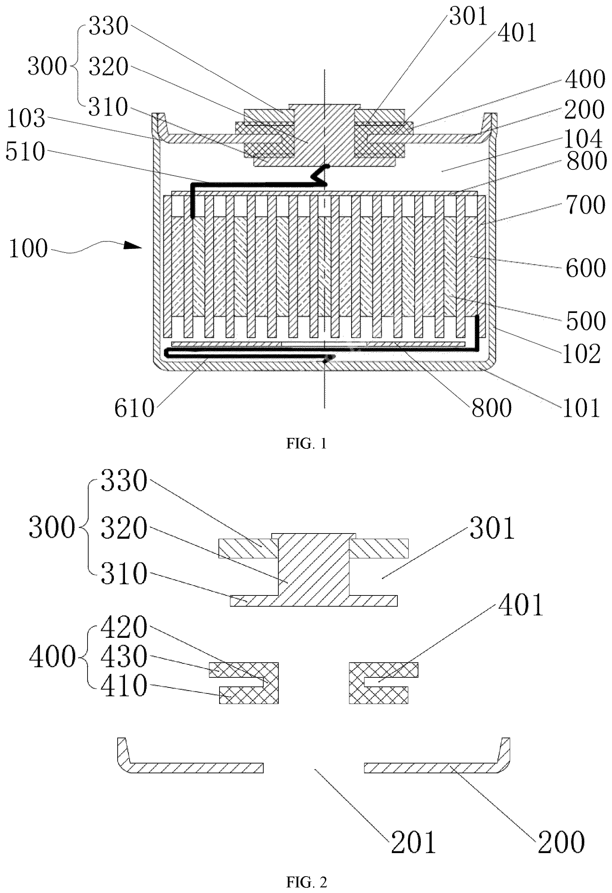

Referring to FIGS. 1-2, a cover plate assembly is provided for a micro battery, which includes a housing 100, with an accommodation cavity 104 defined inside the housing 100 and having an opening 103; the cover plate assembly includes a cover plate 200, an electrode post structure 300, and a sealing ring 400, with the cover plate 200 configured to cover the opening 103 and in sealed cooperation with a side wall of the opening 103, thereby forming a seal between the cover plate 200 and the housing 100. Specifically, the cover plate 200 is welded to the side wall of the opening 103 to form a seal. The electrode post structure 300 passes through the cover plate 200, and an outer peripheral side wall of the electrode post structure 300 defines a sealing ring accommodation groove 301; the sealing ring 400 is disposed on the outer peripheral side wall of the electrode post structure 300 and accommodated within the sealing ring accommodation groove 301, and forms a sealed fit with the sealing ring accommodation groove 301, thereby enhancing the sealing performance between the sealing ring 400 and the electrode post structure 300, and fixing the sealing ring 400 relative to the electrode post structure 300 for easy assembly. An outer peripheral side wall of the sealing ring 400 is in a sealed fit with an inner peripheral side wall of the cover plate 200, thereby enabling the inner and outer sides of the sealing ring 400 to form good seals with the electrode post structure 300 and the cover plate 200, respectively, and thus preventing leakage. Additionally, replacing the traditional glass-sealed structure with the sealing ring 400 may reduce manufacturing complexity and precision control requirements.

In the embodiments, the sealing ring 400 may be made of plastic material to allow it to undergo elastic deformation and achieve good sealing performance. Specifically, the material of the sealing ring 400 may be PP, PPS, GPPS, PFA, HDPE, or PC to enhance the sealing performance of the sealing ring 400. Tests have shown that sealing rings 400 made from these materials can extend the battery's service life and reduce self-discharge rates. Additionally, these materials enable the sealing ring 400 to possess certain insulating properties.

Referring to FIGS. 1-2, a longitudinal cross-sectional shape of the electrode post structure 300 is I-shaped. A recess on the outer peripheral side wall of the electrode post structure 300 forms the sealing ring accommodation groove 301, thereby simplifying the overall structure of the electrode post structure 300. The sealing ring 400 is interference-fit with the sealing ring accommodation groove 301. Therefore, along the central axis of the electrode post structure 300, the length of the sealing ring 400 shall be slightly greater than a distance between two side walls of the sealing ring accommodation groove 301. Since the sealing ring 400 is made of plastic material, it has a certain degree of elasticity, enabling it to assemble with the side walls of the sealing ring accommodation groove 301 and form an interference fit, thereby enhancing the sealing performance between the sealing ring 400 and the side walls of the sealing ring accommodation groove 301.

Referring further to FIGS. 1-2, the cover plate 200 defines a mounting hole 201 extending through its centerline direction. The outer peripheral side wall of the sealing ring 400 defines a cover plate accommodation groove 401, and the sealing ring 400 is accommodated within the mounting hole 201. A side wall of the cover plate accommodation groove 401 is in contact with an outer surface of the cover plate 200, and another side wall of the cover plate accommodation groove 401 is in contact with an inner surface of the cover plate 200, thereby enhancing the sealing performance between the sealing ring and the cover plate and further preventing leakage.

Referring again to FIGS. 1-2, the electrode post structure 300 includes a first pressure plate 310, an electrode post body 320, and a second pressure plate 330. The first pressure plate 310 and the second pressure plate 330 are respectively connected to two ends of the electrode post body 320 along its axis and spaced apart, defining the sealing ring accommodation groove 301; the sealing ring 400 includes a first side wall 410, a bottom wall 420, and a second side wall 430, the first side wall 410 and the second side wall 430 are respectively connected to two ends of the sealing ring bottom wall 420 along its axis and spaced apart, defining the cover plate accommodation groove 401. Along a radial direction of the electrode post structure 300, the distance between an outer wall of the first side wall 410 and an outer wall of the bottom wall 420 is greater than the distance between an outer wall of the first pressure plate 310 and an outer wall of the electrode post body 320, such that the entire surface of the first pressure plate 310 near the first side wall 410 can be in close contact with the first side wall 410, thereby improving the sealing performance. And/or, along the radial direction of the electrode post structure 300, the distance between an outer wall of the second side wall 430 and an outer wall of the bottom wall 420 is greater than the distance between an outer wall of the second pressure plate 330 and an outer wall of the electrode post body 320, such that the entire surface of the second pressure plate 330 near the second side wall 430 can be in close contact with the second side wall 430, thereby improving sealing performance. The above embodiments exemplarily illustrate a configuration where the distance between the outer wall of the first side wall 410 and the outer wall of the bottom wall 420 of the sealing ring is greater than the distance between the outer wall of the first pressure plate 310 and the outer wall of the electrode post body 320, and the distance between the outer wall of the second side wall 430 and the outer wall of the bottom wall 420 of the sealing ring is greater than the distance between the outer wall of the second pressure plate 330 and the outer wall of the electrode post body 320.

Referring again to FIGS. 1-2, the first side wall 410 is disposed inside the accommodation cavity 104, and the second side wall 430 is disposed outside the accommodation cavity 104. Since the second side wall 430 is on a side of the cover plate 200 facing the external surface, it comes into direct contact with the external environment, thus requiring higher sealing performance between the second side wall 430 and the second pressure plate 330. To meet this requirement, in the embodiments, along the radial direction of the electrode post structure 300, the distance between the outer wall of the first side wall 410 and the outer wall of the bottom wall 420 is greater than the distance between the outer wall of the second side wall 430 and the outer wall of the bottom wall 420.

Referring further to FIGS. 1-2, the first pressure plate 310 is disposed inside the accommodation cavity 104, and the second pressure plate 330 is disposed outside the accommodation cavity 104. The first pressure plate 310 is integrally formed with the electrode post body 320, and the second pressure plate 330 is riveted to the electrode post body 320. The sealing ring 400 may first be sleeved on the electrode post body 320, and then pushed such that the sealing ring 400 abuts against the first pressure plate 310. Subsequently, the second pressure plate 330 may be mounted on the electrode post body 320 via riveting to complete the assembly.

The embodiments further provide a micro battery, including the aforementioned cover plate assembly, as well as a housing 100 and a cell structure. The housing 100 includes a bottom wall 101 and a side wall 102 surrounding the bottom wall 101. The side wall 102 and the bottom wall 101 together define the accommodation cavity 104 with an opening 103 at one end. The diameter of the housing 100 does not exceed 10 mm, thereby minimizing the size of the micro battery. The cover plate 200 is configured to cover and seal the opening 103, and the cell structure is disposed within the accommodation cavity 104. **In** the cover plate assembly, the cover plate 200 is sealed to the housing 100, and the inner and outer sides of the sealing ring 400 can form a good seal with the electrode post structure 300 and the cover plate 200, respectively, to prevent leakage. Additionally, replacing the traditional glass-sealed structure with the sealing ring 400 may reduce manufacturing complexity and precision control requirements.

Referring to FIGS. 1-2, the cell structure includes a positive electrode sheet 500 and a negative electrode sheet 600. The positive electrode sheet 500 is electrically connected to the electrode post structure 300, and the negative electrode sheet 600 is electrically connected to the housing 100. A positive terminal of the external structure can be connected to the electrode post assembly 300, and a negative terminal can be directly connected to the housing 100, enabling the battery to supply power to the external structure. **In** the embodiments, the external structure serves as a working module of the capsule endoscope. The positive electrode sheet 500 and the negative electrode sheet 600 are wound together to form a jellyroll, with the outermost layer being the negative electrode sheet 600, thereby constituting a wound battery. Since the negative electrode sheet 600 is electrically connected to the housing 100, the outermost layer being the negative electrode sheet 600 may facilitate connection between the negative electrode sheet 600 and the housing 100 while preventing contact between the positive electrode sheet 500 and the housing 100, thereby avoiding short circuits and ensuring safety. Additionally, since the charge capacity of the micro battery depends on the size of the positive electrode sheet 500, positioning the negative electrode sheet 600 on the outermost layer to enclose the positive electrode sheet 500 may ensure that the performance of the entire positive electrode sheet 500 is fully utilized. Compared to a design where the positive electrode sheet 500 is positioned on the outermost layer, this configuration may enhance the energy density of the micro battery.

Referring to FIGS. 1-2, the peripheral length of the outermost layer of the negative electrode sheet 600 is L. The outermost layer of the negative electrode sheet 600 extends along the periphery after covering the outermost layer of the positive electrode sheet 500, with an extension length being no less than L/5, thereby ensuring that the outermost layer of the negative electrode sheet 600 fully covers the outermost layer of the positive electrode sheet 500.

Referring to FIGS. 1-2, the height of the housing 100 does not exceed 10 mm, and the diameter of the housing 100 also does not exceed 10 mm, which reduces the size of the housing 100, enabling it to fit into a narrow space of the capsule endoscope. In some embodiments, the diameter of the housing 100 ranges from 6 mm to 10 mm, specifically 6 mm, 6.5 mm, 7 mm, 7.5 mm, 8 mm, 8.5 mm, 9 mm, 9.5 mm, or 10 mm; the height of the housing 100 ranges from 5 mm to 10 mm, specifically 5 mm, 5.5 mm, 6 mm, 6.5 mm, 7 mm, 7.5 mm, 8 mm, 8.5 mm, 9 mm, 9.5 mm, or 10 mm.

Referring again to FIGS. 1-2, a surface of the positive electrode sheet 500 is coated with an electrode active material, which serves to enhance battery performance, control electrode thickness, ensure battery safety, and optimize battery manufacturing processes. In the embodiments, the thickness of the positive electrode sheet 500 after coating with the electrode active material is 0.2 mm to 0.5 mm, and the length is 50 mm to 300 mm. Tests have shown that using a thicker electrode may enable the battery's 2C continuous discharge capacity to exceed 80%.

Referring further to FIGS. 1-2, an insulating layer 700 is arranged between the positive electrode sheet 500 and the negative electrode sheet 600 to prevent contact between the positive electrode sheet 500 and the negative electrode sheet 600. To ensure insulation effectiveness, along the centerline direction of the jellyroll, i.e., the direction of the centerline where the positive electrode sheet 500 and the negative electrode sheet 600 are wound together, the length of the positive electrode sheet 500 is the same as that of the negative electrode sheet 600, while the length of the insulating layer 700 is greater than the length of the positive electrode sheet 500 and the length of the negative electrode sheet 600. Additionally, both ends of the insulating layer 700 extend beyond the positive electrode sheet 500 or the negative electrode sheet 600.

Referring further to FIGS. 1-2, along the centerline direction of the jellyroll, at least one end of the jellyroll is arranged with an insulating spacer 800. In the embodiments, along the centerline direction of the jellyroll, the jellyroll is arranged with insulating spacers 800 on both ends. The insulating spacers 800 prevent the jellyroll from coming into direct contact with other structures along its centerline direction.

The embodiments of the present disclosure further provide a capsule endoscope, which includes the aforementioned micro battery, an endoscope housing, and a working module. The micro battery and the working module are both disposed inside the endoscope housing, and the micro battery is electrically connected to the working module. The micro battery has a small size, and the cover plate assembly has good sealing performance with the housing 100, thereby preventing leakage. Additionally, the use of the sealing ring replaces the traditional glass-sealed structure, thereby reducing manufacturing complexity and precision control requirements.

## Claims

1. A cover plate assembly for a micro battery; wherein the micro battery comprises a housing (100), with an accommodation cavity (104) defined inside the housing (100) and having an opening (103); the cover plate assembly comprises:
a cover plate (200), configured to cover and seal the opening (103) and in sealed cooperation with a side wall of the opening (103);
an electrode post structure (300), passing through the cover plate (200); wherein an outer peripheral side wall of the electrode post structure (300) defines a sealing ring accommodation groove (301); and
a sealing ring (400), disposed on the outer peripheral side wall of the electrode post structure (300) and accommodated within the sealing ring accommodation groove (301); wherein the sealing ring (400) is in sealed cooperation with the sealing ring accommodation groove (301).

2. The cover plate assembly according to claim 1, wherein a longitudinal cross-sectional shape of the electrode post structure (300) is I-shaped; a recess on the outer peripheral side wall of the electrode post structure (300) forms the sealing ring accommodation groove (301), and the sealing ring (400) is interference-fit with the sealing ring accommodation groove (301).

3. The cover plate assembly according to claim 1, wherein the cover plate (200) defines a mounting hole (201) extending through a centerline direction of the cover plate (200); the outer peripheral side wall of the sealing ring (400) defines a cover plate accommodation groove (401), and the sealing ring (400) is accommodated within the mounting hole (201); a side wall of the cover plate accommodation groove (401) is in contact with an outer surface of the cover plate (200), and another side wall of the cover plate accommodation groove (401) is in contact with an inner surface of the cover plate (200).

4. The cover plate assembly according to claim 3, wherein the electrode post structure (300) comprises a first pressure plate (310), an electrode post body (320), and a second pressure plate (330); the first pressure plate (310) and the second pressure plate (330) are connected to two ends of the electrode post body (320) along an axial direction of the electrode post body (320) and spaced apart, defining the sealing ring accommodation groove (301); the sealing ring (400) comprises a first side wall (410), a bottom wall (420), and a second side wall (430), the first side wall (410) and the second side wall (430) are connected to two ends of the bottom wall (420) along an axial direction of the bottom wall (420) and spaced apart, defining the cover plate accommodation groove (401);
along a radial direction of the electrode post structure (300), a distance between an outer wall of the first side wall (410) and an outer wall of the bottom wall (420) is greater than a distance between an outer wall of the first pressure plate (310) and an outer wall of the electrode post body (320); and/or
along a radial direction of the electrode post structure (300), a distance between an outer wall of the second side wall (430) and an outer wall of the bottom wall (420) is greater than a distance between an outer wall of the second pressure plate (330) and an outer wall of the electrode post body (320).

5. The cover plate assembly according to claim 4, wherein the first side wall (410) is disposed inside the accommodation cavity (104), and the second side wall (430) is disposed outside the accommodation cavity (104); along the radial direction of the electrode post structure (300), the distance between the outer wall of the first side wall (410) and the outer wall of the bottom wall (420) is greater than the distance between the outer wall of the second side wall (430) and the outer wall of the bottom wall (420).

6. The cover plate assembly according to any one of claims 2-5, wherein the electrode post structure (300) comprises a first pressure plate (310), an electrode post body (320), and a second pressure plate (330); the first pressure plate (310) and the second pressure plate (330) are connected to two ends of the electrode post body (320) along an axial direction of the electrode post body (320) and spaced apart, defining the sealing ring accommodation groove (301); the first pressure plate (310) is disposed inside the accommodation cavity (104), and the second pressure plate (330) is disposed outside the accommodation cavity (104); the first pressure plate (310) is integrally formed with the electrode post body (320), and the second pressure plate (330) is riveted to the electrode post body (320).

7. A micro battery, comprising the cover plate assembly according to any one of claims 1-6, the housing (100), and a cell structure; wherein the housing (100) comprises a bottom wall (101) and a side wall (102) surrounding the bottom wall (101); the side wall (102) and the bottom wall (101) together define the accommodation cavity (104) with the opening (103) at one end; a diameter of the housing (100) is greater than or equal to 10 mm; the cover plate (200) is configured to cover and seal the opening (103), and the cell structure is disposed within the accommodation cavity (104).

8. The micro battery according to claim 7, wherein the cell structure comprises a positive electrode sheet (500) and a negative electrode sheet (600); the positive electrode sheet (500) is electrically connected to the electrode post structure (300), and the negative electrode sheet (600) is electrically connected to the housing (100); the positive electrode sheet (500) and the negative electrode sheet (600) are wound together to form a jellyroll, with an outermost layer being the negative electrode sheet (600).

9. The micro battery according to claim 8, wherein a peripheral length of an outermost layer of the negative electrode sheet (600) is L; the outermost layer of the negative electrode sheet (600) extends along a periphery of the jellyroll after covering an outermost layer of the positive electrode sheet (500), with an extension length being no less than L/5.

10. A capsule endoscope, comprising the micro battery according to any one of claims 7-9, an endoscope housing, and a working module; wherein the micro battery and the working module are both disposed inside the endoscope housing, and the micro battery is electrically connected to the working module.
